# EUROPÄISCHE PATENTSCHRIFT

(11) **EP 0 784 642 B1**
(45) Veröffentlichungstag und Bekanntmachung des Hinweises auf die Patenterteilung: **10.03.1999**
(21) Anmeldenummer: 95934660.2
(22) Anmeldetag: 06.10.1995
(51) Int. Cl.: C08G 65/30, C08G 63/90, C07C 41/34

(54) **VERFAHREN ZUR ABTRENNUNG VON HETEROPOLYVERBINDUNGEN AUS POLYETHERN, POLYESTERN UND POLYETHERESTERN**
PROCESS FOR EXTRACTING HETEROPOLYCOMPOUNDS FROM POLYETHERS, POLYESTERS AND POLYETHER ESTERS
PROCEDE D'EXTRACTION DES HETEROPOLYCOMPOSES CONTENUS DANS DES POLYETHERS, POLYESTERS ET ESTERS DE POLYETHERS

(30) Priorität: 07.10.1994 DE 4435920
(43) Veröffentlichungstag der Anmeldung: 23.07.1997
(73) Patentinhaber: BASF AKTIENGESELLSCHAFT, 67056 Ludwigshafen (DE)
(72) Erfinder: WEYER, Hans-Jürgen, D-67240 Bobenheim-Roxheim (DE); FISCHER, Rolf, D-69121 Heidelberg (DE); JESCHEK, Gerhard, D-67269 Grünstadt (DE); MÜLLER, Herbert, D-67227 Frankenthal (DE)
(74) Vertreter: Isenbruck, Günter, Dr.
(86) Internationale Anmeldenummer: EP9503955
(87) Internationale Veröffentlichungsnummer: WO9611221

(56) Entgegenhaltungen:
- EP-A- 0 158 229
- EP-A- 0 428 003
- EP-A- 0 503 393
- DD-A- 38 244
- US-A- 4 946 939
- US-A- 4 952 668

## Beschreibung

Die Erfindung betrifft ein Verfahren zur Abtrennung von Heteropolyverbindungen aus Gemischen, die Polyether, Polyester und/oder Polyetherester, eine protische Komponente und Heteropolyverbindungen enthalten, indem die protische Komponente aus dem Gemisch entfernt und die Heteropolyverbindung danach als gesonderte Phase abgetrennt wird.

Polyether, Polyester und Polyetherester werden vielseitig verwendet, beispielhaft sei hier deren Verwendung in Hydraulikölen oder als Diolkomponente bei der Herstellung von Polyurethanen genannt. Diese Verbindungen werden durch kationische Polymerisation oder Copolymerisation entsprechender Monomere, wie cyclischer Ether, Acetale, Polyalkohole oder lactone, mit Hilfe von Brönstedt- oder Lewis-Säure-Katalysatoren hergestellt. Als besonders vorteilhafte Katalysatoren für eine Ringöffnungspolymerisation haben sich Heteropolysäuren und Heteropolysäuresalze, im folgenden gemeinsam als "Heteropolyverbindung(en)" oder "HPA" bezeichnet, erwiesen. Bei verschiedenen Verfahren zur Herstellung bestimmter neuer Derivate, aber auch zur Verbesserung der technischen Durchführbarkeit und der Produktqualität bei bestehenden Verfahren, wird die Polymerisation in Gegenwart protischer Verbindungen durchgeführt.

EP 126 471 lehrt beispielsweise die HPA-katalysierte Polymerisation von Tetrahydrofuran (THF) und die Copolymerisation von THF mit verschiedenen anderen cyclischen Ethern in Gegenwart von Wasser unter Bildung von Polyetherglykolen. In EP 158 229 wird die Herstellung von Polyetherglykolen durch Copolymerisation cyclischer Ether mit di- und höherfunktionellen Alkoholen beschrieben.

Nach JP 61-200120 können Lactone oder Lactone zusammen mit cyclischen Ethern in Gegenwart von Hydroxylgruppen-haltigen Verbindungen mit Heteropolysäuren als Katalysatoren polymerisiert werden.

Polyetherglykolmonoether und Polyetherglykolmonoester lassen sich nach EP 503 393 und EP 503 394 durch Polymerisation cyclischer Ether in Gegenwart von Monoalkoholen oder Monocarbonsäuren mit HPA-Katalysatoren herstellen.

Bei diesen Polymerisationsverfahren zur Herstellung von Polyethern, Polyestern und Polyetherestern entstehen Polymerphasen, die aufgrund des unvollständigen Umsatzes noch verbleibende Mengen an Monomer(en), verbleibende Mengen an zugesetzter protischer Verbindung wie Wasser, Alkoholen, Carbonsäuren usw., gegebenenfalls eingesetzte Lösungsmittel sowie gelöste Heteropolyverbindungen(en) enthalten. Der Prozentsatz an in der Polymerphase gelöstem HPA-Katalysator ist bei diesen Reaktionen beträchtlich und kann bis zu 1 Gew.-% und mehr, bezogen auf die Polymerphase, betragen. Der gelöste Katalysator fällt bei einer Abtrennung, die lediglich aus einem Abdestillieren von unumgesetztem Monomer, protischem Zusatz und gegebenenfalls eingesetztem Lösungsmittel aus dieser Phase besteht, nicht aus, sondern verbleibt in gelöster Form im Polymer. Einerseits muß dies aus Qualitätsgründen verhindert werden, andererseits ist es aus Kostengründen, da Heteropolysäuren sehr teuer sind, wünschenswert, den größten Teil des Katalysators wiederzugewinnen.

In EP 181 621 wird zur Lösung dieses Problems vorgeschlagen, die Polymerphase mit einem Kohlenwasserstoff oder halogeniertem Kohlenwasserstoff, der mit dem bzw. den verwendeten Monomer(en) kein Azeotrop bildet, zu versetzen, wodurch der größte Teil der gelösten Heteropolyverbindung als gesonderte Phase ausgefällt wird. Anschließend wird die Kohlenwasserstoff/Polymer-Phase auf an sich übliche Weise mit einem festen Adsorbens behandelt. Zur Gewinnung des Polymers müssen zum Schluß nach Abtrennung von unumgesetztem Monomer, protischem Zusatz und gegebenenfalls zugesetztem Lösungsmittel die zuletzt zugegebenen (gegebenenfalls halogenierten) Kohlenwasserstoffe abgetrennt werden. Ein solches Aufarbeitungsverfahren weist den Nachteil auf, daß wegen des durch die Zugabe des (gegebenenfalls halogenierten) Kohlenwasserstoffs vergrößerten Volumens der Polymerphase größer dimensionierte Apparate zur Destillation erforderlich sind und somit höhere Investitionskosten anfallen, wobei durch die Entfernung des zugesetzten (ggf. halogenierten) Kohlenwasserstoffs, dessen Siedepunkt gewöhnlich über demjenigen des bzw. der Monomeren liegt, beträchtliche zusätzliche Energiekosten entstehen.

In SU 1754732 A1 wird nach Konzentration der Polymerphase auf 50 - 90 % Polymer ebenfalls ein Kohlenwasserstoff mit 1 - 15 C-Atomen zur Ausfällung der Heteropolyverbindung zugesetzt. Anstelle einer weiteren Reinigung durch Adsorption wird dann jedoch eine flüssige organische Stickstoffbase zugesetzt, die mit noch vorhandener Heteropolyverbindung, bei der es sich in diesem Fall um die Säure handeln muß, ein unlösliches Salz bildet, das ausfällt und auf übliche Weise abgetrennt werden kann. Die somit zu erzielende Reinheit von ca. 50 x 10⁻⁶% Heteropolyverbindung im Polymer ist jedoch für die meisten Zwecke nicht ausreichend.

Aufgabe der vorliegenden Erfindung war es demgemäß, ein wirkungsvolles einfacheres und kostengünstigeres Verfahren zur Reinigung von Heteropolysäuren enthaltenden Polyethern, Polyestern und Polyetherestern zu schaffen.

Gelöst wird diese Aufgabe durch ein Verfahren zur Abtrennung von Heteropolyverbindungen aus Gemischen, die Polyether, Polyester und/oder Polyetherester, eine protische Komponente und Heteropolyverbindungen enthalten, dadurch gekennzeichnet, daß die protische Komponente mit Hilfe eines Membran-Trennverfahrens oder durch Destillation, gegebenenfalls azeotrop zusammen mit gegebenenfalls vorhandenem unumgesetztem Monomer und/oder Lösungsmittel, aus dem Gemisch abgetrennt wird, daß unumgesetztes Monomer und gegebenenfalls vorhandenes Lösungsmittel vollständig abdestilliert werden und die Heteropolyverbindungen danach durch Zugabe des besagten Monomers in im wesentlichen reiner Form ausgefällt wird.

Nach dem erfindungsgemäßen Verfahren können Polyether, Polyester und/oder Polyetherester von Heteropolyverbindung(en) gereinigt werden. Dabei kann das beschriebene Reinigungsverfahren für jedes Gemisch, das HPA und eines oder mehrere derartiger Polymere enthält, eingesetzt werden. Die genannten polymeren Verbindungen können beispielsweise aus Monomeren der Gruppe der cyclischen Ether, Acetale, Diole, höheren Alkohole und Lactone aufgebaut sein. Es ist aber auch möglich, aus anderen Monomeren bestehende polymere Verbindungen nach dem erfindungsgemäßen Verfahren von gelösten Heteropolyverbindungen zu befreien. Die Molmasse der zu reinigenden Polyether, Polyester und/oder Polyetherester ist nicht beschränkt, liegt aber bevorzugt unter 5000.

Man setzt die Heteropolyverbindung(en) enthaltenden Polyether, Polyester und/oder Polyetherester als solche oder in gelöster Form, beispielsweise und bevorzugt in ihrer bei der Polymerisation anfallenden Lösung, ein. In dieser Beschreibung und den Ansprüchen wird als "Polymerphase" oder "(Polymer)gemisch" eine Phase bezeichnet, die mindestens eines der obigen Polymere enthält, während als "Polymerlösung" eine Phase bezeichnet wird, die noch zusätzlich eine als Lösungsmittel wirkende niedermolekulare organische Komponente enthält.

Das Polymerisationsverfahren selbst spielt für die erfindungsgemäße Reinigung von Polyethern, Polyestern und/oder Polyetherestern keine besondere Rolle. Die Polymerisation kann kontinuierlich oder diskontinuierlich durchgeführt werden, und das Polymerisationssystem kann einphasig, zweiphasig oder heterogen sein.

In der Regel enthält die bei der Polymerisation gebildete Polymerlösung noch einen beträchtlichen Anteil an Monomer(en), einen großen Teil der anfangs zugesetzten protischen Verbindung(en) sowie gelöste Heteropolyverbindung(en). Unter protischer Verbindung wird für die Zwecke dieser Erfindung eine Verbindung verstanden, die OH-Gruppen aufweist und einen pKs ≤ 20 besitzt; Beispiele sind H₂O, Carbonsäuren und Alkohole. Da es vorteilhaft sein kann, die Polymerisation in Gegenwart eines Lösungsmittels durchzuführen, kann das Polymerisationssystem gegebenenfalls auch ein solches enthalten.

In einem bevorzugten Polymerisationsverfahren mit HPA-Katalyse wird so gearbeitet, daß ein Polymerisationsgemisch mit zwei flüssigen Phasen entsteht, wobei die eine die Katalysatorphase und die andere die Monomer/Polymer-Phase ist. Eine typische Monomer-/Polymer-Phase, wie sie beispielsweise bei der Ringöffnungspolymerisation von THF zu Polytetrahydrofuran (PolyTHF) in Gegenwart von Wasser mit H₃PW₁₂O₄₀ als Katalysator in einem zweiphasigen Polymerisationssystem entsteht, ist wie folgt zusammengesetzt (Angaben in Gew.-%): 77,3% THF, 21,2% PolyTHF, 1,2% H₃PW₁₂O₄₀, 0,3% Wasser.

Die Zusammensetzung der Polymerphase, die für das erfindungsgemäße Verfahren verwendet wird, hängt demgemäß von der Art des Katalysators, des oder der Monomeren, des protischen Zusatzes und dem Gehalt von möglicherweise verwendetem Lösungsmittel ab und ist für das Verfahren nicht kritisch.

Die erfindungsgemäße Entfernung protischer Verbindungen aus den Polymerisationslösungen kann durch verschiedenste Methoden erfolgen. Beispielsweise kann es günstig sein, Membrantrennverfahren einzusetzen. Weiterhin können protische Verbindungen durch Adsorption, beispielsweise mit Hilfe von Molekularsieben, oder durch Absorption, beispielsweise durch Entfernung mittels einer chemischen Reaktion, abgetrennt werden. Ein apparativ sehr einfaches Verfahren ist die destillative Abtrennung der protischen Verbindung, ggf. als Azeotrop mit Monomer. Man kann auch alle leichtsiedenden Komponenten zusammen aus der Polymerlösung entfernen und anschließend wieder eine entsprechende Menge an reinem Monomer, das im wesentlichen frei von protischen Verbindungen ist, zusetzen. Besonders bevorzugt wird bei der destillativen Abtrennung in einem Druckbereich gearbeitet, der eine Siedetemperatur von 50 - 70 °C ermöglicht.

Beim Entfernen der protischen Verbindung oder, falls alle leichtsiedenden Komponenten zusammen entfernt werden, bei der erneuten Zugabe von reinem Monomer fällt der größte Teil der in der Polymerlösung gelösten HPA in fester Form aus. Dabei kann es vorteilhaft sein, die Lösung auf geeignete Weise, beispielsweise durch konventionelles Rühren, zu durchmischen. Für die HPA-Entfernung werden Temperaturen gewählt, bei denen das Verfahren einfach durchzuführen ist, normalerweise Temperaturen zwischen 0 und 60 °C; bei höheren Temperaturen kann die HPA-Löslichkeit in der Polymerphase zu groß sein. Die Mischung kann zur Vervollständigung der Fällung 0,1 bis 100 Stunden stehenbleiben. Üblicherweise genügen höchstens 0,5 Stunden.

Im Fall einer destillativen Abtrennung der protischen Verbindung wird die Menge des abdestillierten und/oder zugegebenen Monomers geeigneterweise für jeden Einzelfall optimiert. Im allgemeinen ist es zum Ausfällen der Heteropolyverbindung nach Abtrennung der protischen Verbindung günstig, wenn ein Monomer-Polymer-Massenverhältnis von 0,1:1 bis 10:1 eingestellt wird.

Das abgetrennte Azeotrop aus Monomer und protischer Verbindung oder auch die Mischung aller abgetrennten leichtflüchtigen Verbindungen kann normalerweise ohne weitere Aufarbeitung direkt in die Polymerisationsstufe zurückgeführt werden.

Das erfindungsgemäße Verfahren kann kontinuierlich oder diskontinuierlich, bevorzugt jedoch kontinuierlich, durchgeführt werden, wobei vorteilhafterweise unter Inertgasatmosphäre gearbeitet wird. Herkömmliche, für kontinuierliche Verfahren geeignete Reaktoren oder Reaktoranordnungen, beispielsweise Rohrreaktoren oder Rührkesselkaskaden, können dafür verwendet werden.

Durch das erfindungsgemäße Verfahren gelingt die Abtrennung des größten Teils der in den Polyether-, Polyester- und/oder Polyetherester-Lösungen gelösten HPA ebenfalls in einer Form, die direkt in die Polymerisationsstufe zurückgeführt werden kann. Beispielsweise können durch Abdestillieren von unumgesetztem THF und Wasser aus der oben erwähnten Monomer/Polymerphase, die bei einer H₃PW₁₂O₄₀-katalysierten THF-Polymerisation in Gegenwart von Wasser erhalten worden ist, und anschließenden Ersatz des abdestillierten THF durch reines, trockenes THF etwa 90% des ursprünglich in der THF/PolyTHF-Phase gelösten H₃PW₁₂O₄₀ in fester Form abgetrennt werden.

Die einzelnen bei der HPA-Ausfällung durch Entfernung protischer Verbindungen ablaufenden Vorgänge sind noch unklar. Eine mögliche zugrunde liegende Tatsache könnte sein, daß die Entfernung der protischen Komponenten durch den Wegfall von Wasserstoffbrückenbindungen die Löslichkeit der Heteropolyverbindungen so stark herabsetzt, daß diese aus der Polymerlösung ausfallen.

Unter Heteropolysäuren im Sinne der vorliegenden Erfindung werden anorganische Polysäuren mit mindestens zwei verschiedenen Zentralatomen verstanden, welche aus schwachen, mehrbasigen Sauerstoffsäuren eines Metalls, vorzugsweise aus denen des Chroms, Molybdäns, Vanadiums und Wolframs, und/oder den entsprechenden Oxiden dieser Metalle, beispielsweise CrO₃, MoO₃, V₂O₅ oder WO₃, und denen eines anderen Metalls oder Nichtmetalls, beispielsweise Arsen, Bor, Iod, Phosphor, Selen, Silicium, Germanium oder Tellur, als gemischte, partielle Anhydride entstehen. In der Regel hat das Atomverhältnis zwischen den erstgenannten und den letztgenannten Elementen in diesen Heteropolysäuren den Wert 2,5 bis 12, vorzugsweise beträgt das Atomverhältnis 9 oder 12.

Als Beispiele für Heteropolysäuren, wie sie im erfindungsgemäßen Verfahren entfernt werden können, seien die folgenden Verbindungen genannt:
Dodecamolybdatophosphorsäure (H₃PMo₁₂O₄₀ x nH₂O),
Dodecamolybdatokieselsäure (H₄SiMo₁₂O₄₀ x nH₂O),
Dodecamolybdatocer(IV)säure (H₈CeMo₁₂O₄₂ x nH₂O),
Dodecamolybdatoarsen(V)säure (H₃AsMo₁₂O₄₀ x nH₂O),
Hexamolybdatochrom(III)säure (H₃CrMO₆O₂₄H₆ x nH₂O),
Hexamolybdatonickel(II)säure (H₄NiMO₆O₂₄H₆ x 5H₂O),
Hexamolybdatoiodsäure (H₅IMO₆O₂₄ x nH₂O),
Octadecamolybdatodiphosphorsäure (H₆P₂Mo₁₈O₆₂ x 11H₂O),
Octadecamolybdatodiarsen(V)säure (H₆As₂Mo₁₈O₆₂ x 25H₂O),
Nonamolybdatomangan(IV)säure (H₆MnMO₉O₃₂ x nH₂O),
Undecamolybdatovanadatophosphorsäure (H₄PMo₁₁VO₄₀ x nH₂O),
Decamolybdatodivanadatophosphorsäure (H₅PMo₁₀V₂O₄₀ x nH₂O),
Dodecavanadatophosphorsäure (H₇PV₁₂O₃₆ x nH₂O),
Dodecawolframatokieselsäure (H₄SiW₁₂O₄₀ x 7H₂O),
Dodecawolframatophosphorsäure (H₃PW₁₂O₄₀ x nH₂O),
Dodecawolframatoborsäure (H₅BW₁₂O₄₀ x nH₂O),
Octadecawolframatodiphosphorsäure (H₆P₂W₁₈O₆₂ x 14H₂O),
Octadecawolframatodiarsen(V)säure (H₆As₂W₁₈O₆₂ x 14H₂O),
Hexamolybdatohexawolframatophosphorsäure (H₃PMo₆W₆O₄₀ x nH₂O).

Selbstverständlich können auch Mischungen von Heteropolysäuren entfernt werden. Häufig dient das erfindungsgemäße Verfahren zur Entfernung von Dodecawolframatophosphorsäure, Dodecawolframatokieselsäure und/oder Dodecamolybdatokieselsäure, da diese aufgrund ihrer guten Verfügbarkeit bevorzugt als Katalysatoren eingesetzt werden.

Das erfindungsgemäße Verfahren wird besonders bevorzugt für die Reinigung von Polymeren verwendet, bei deren Herstellungsverfahren die freien Heteropolysäuren als Katalysatoren eingesetzt worden sind. Aber auch die Abtrennung von Alkali- und/oder Erdalkalimetallsalzen von Heteropolysäuren ist möglich.

Polyether, Polyester und Polyetherester mit einem wie vorstehend beschrieben verringerten HPA-Gehalt können durch Inkontaktbringen mit einem festen Adsorbens weiter gereinigt werden. Dabei können diese Polymere direkt oder in Lösung mit dem festen Adsorbens behandelt werden. Bevorzugt ist der Einsatz von gegebenenfalls noch lösungsmittelhaltigen Monomer/Polymer-Mischungen. Dabei können diese Mischungen, so wie sie bei der HPA-Ausfällung anfallen, aber auch in konzentrierterer oder verdünnter Form eingesetzt werden. Die in der Polymerphase vorteilhafterweise enthaltene Monomermenge sollte mindestens 10 Gew.-%, besser 50 Gew.-% und mehr, betragen.

Die Art des festen Adsorbens ist nicht beschränkt, solange es Heteropolyverbindungen adsorbieren kann; bevorzugt sind Aktivkohlen, Aluminiumoxide, Oxide, Hydroxide und Carbonate von Erdalkali- und Seltenerdmetallen sowie basische Ionenaustauscher. Die Menge des verwendeten Adsorbens hängt vom HPA-Gehalt ab und kann das 2- bis 5000fache, bevorzugt das 10- bis 1000fache, an gelöster HPA betragen. Im allgemeinen führt der Einsatz größerer Mengen an festem Adsorbens zu einem geringeren HPA-Restgehalt nach der Behandlung.

Die Temperatur bei diesem Reinigungsschritt ist nicht besonders beschränkt und sollte so gewählt werden, daß die zu behandelnde Lösung eine geeignete Viskosität aufweist. Wird ein Polyether mit mittlerer Molmasse 1000 in reiner Form eingesetzt, liegt die geeignete Temperatur normalerweise zwischen 20 und 150 °C, bevorzugt zwischen 30 und 100 °C.

Wenn das gereinigte Polymer nach der Behandlung mit einem festen Adsorbens noch Monomer oder Lösungsmittel enthält, können diese beispielsweise durch Destillation bei Normaldruck oder unter vermindertem Druck entfernt werden, wobei ein Polymer mit sehr geringem HPA-Gehalt erhalten werden kann, der unter 1 ppm, bezogen auf reinen Polyether, Polyester und/oder Polyetherester, liegen kann. Demgemäß können nach dem erfindungsgemäßen Verfahren Polyether, Polyester und/oder Polyetherester auf wirtschaftliche Weise in hoher Reinheit erhalten werden.

Alle Konzentrationsangaben der folgenden erläuternden Beispiele sind in Gew.-% angegeben. Die Versuche wurden alle unter Stickstoff durchgeführt. Die quantitative Analyse der Heteropolysäuren wurde mittels Röntgenfluoreszenz und Atomabsorption durchgeführt.

### Beispiel 1

Es wurde eine nach EP 126 471 bei der Polymerisation von Tetrahydrofuran (THF) zu Polytetrahydrofuran (PolyTHF) in Gegenwart von Wasser mit H₃PW₁₂O₄₀ als Katalysator anfallende Polymerphase eingesetzt, die die folgende Zusammensetzung aufwies: THF (77,3%), PolyTHF (21,2%), H₃PW₁₂O₄₀ (1,2%), Wasser (0,3%).

200 g dieser Mischung wurden bei 60 °C und 20 mbar von insgesamt 151 g Wasser und THF befreit. Es verblieb ein homogener Rückstand mit klarer, viskoser Konsistenz. Hierzu wurden 150 g wasserfreies THF gegeben, wobei der größte Teil der vorher gelösten H₃PW₁₂O₄₀ ausfiel. Nach Abtrennung von 2,2 g H₃PW₁₂O₄₀ durch Filtration wurde die Polymerphase mit 20 g Aktivkohle (Merck) versetzt und 4 Stunden bei Raumtemperatur geschüttelt. Im nach Abtrennung der Aktivkohle und Einengung im Vakuum erhaltenen PolyTHF lag der H₃PW₁₂O₄₀-Gehalt unter 1 ppm.

### Beispiel 2

Es wurde eine nach EP 503 393 bei der Polymerisation von THF zu Polytetrahydrofuranmonomethylether (PolyTHFmonomethylether) in Gegenwart von Methanol mit H₄SiW₁₂O₄₀ als Katalysator anfallende Polymerphase eingesetzt, die die folgende Zusammensetzung aufwies: THF (84,4%), PolyTHFmonomethylether (11,6%), H₄SiW₁₂O₄₀ (2,2%), Methanol (1,8%).

200 g dieser Mischung wurden bei 60 °C und 20 mbar von insgesamt 170 g THF und Methanol befreit. Es verblieb ein homogener Rückstand mit klarer, viskoser Konsistenz. Hierzu wurden 150 g wasserfreies THF gegeben, wobei der größte Teil der vorher gelösten H₄SiW₁₂O₄₀ ausfiel. Nach Abtrennung von 3,8 g H₄SiW₁₂O₄₀ durch Filtration wurde die Polymerphase mit 20 g Aktivkohle (Merck) versetzt und 4 Stunden bei Raumtemperatur geschüttelt. Im nach Abtrennung der Aktivkohle und Einengung im Vakuum erhaltenen PolyTHFmonomethylether lag der H₄SiW₁₂O₄₀-Gehalt unter 1 ppm.

### Beispiel 3

Es wurde eine nach EP 503 394 bei der Polymerisation von THF zu Polytetrahydrofuranmonoformiat (PolyTHFmonoformiat) in Gegenwart von Ameisensäure mit H₃PW₁₂O₄₀ als Katalysator anfallende Polymerphase eingesetzt, die die folgende Zusammensetzung aufwies: THF (77,1%), PolyTHFmonoformiat (17,6%), H₃PW₁₂O₄₀ (2,4%), Ameisensäure (2,9%).

200 g dieser Mischung wurden bei 60 °C und 20 mbar von insgesamt 155 g Ameisensäure und THF befreit. Es verblieb ein homogener Rückstand mit klarer, viskoser Konsistenz. Hierzu wurden 100 g wasserfreies THF gegeben, wobei der größte Teil der gelösten H₃PW₁₂O₄₀ ausfiel. Nach Abtrennung von 4,2 g H₃PW₁₂O₄₀ durch Filtration wurde die Polymerphase mit 20 g Aktivkohle (Merck) versetzt und 4 Stunden bei Raumtemperatur geschüttelt. Im nach Abtrennung der Aktivkohle und Einengung im Vakuum erhaltenen PolyTHFmonoformiat lag der H₃PW₁₂O₄₀-Gehalt unter 1 ppm.

### Beispiel 4

Es wurde eine nach EP 158 229 bei der Copolymerisation von THF und Ethylenglykol mit H₃PMo₁₂O₄₀ als Katalysator anfallende Polymerphase eingesetzt, die die folgende Zusammensetzung aufwies: THF (82,1%), Copolymer (15,8%), H₃PMo₁₂O₄₀ (1,6%), Ethylenglykol (0,5%).

200 g dieser Mischung wurden bei 60 °C und 20 mbar von insgesamt 162 g THF und Ethylenglykol befreit. Es verblieb ein homogener Rückstand mit klarer, viskoser Konsistenz. Hierzu wurden 150 g wasserfreies THF gegeben, wobei der größte Teil der gelösten H₃PMo₁₂O₄₀ ausfiel. Nach Abtrennung von 2,5 g H₃PMo₁₂O₄₀ durch Filtration wurde die Polymerphase mit 20 g Aktivkohle (Merck) versetzt und 4 Stunden bei Raumtemperatur geschüttelt. Im nach Abtrennung der Aktivkohle und Einengung im Vakuum erhaltenen Copolymer von THF und Ethylenglykol lag der H₃PMo₁₂O₄₀-Gehalt unter 1 ppm.

### Beispiel 5

Es wurde eine nach JP 61/200120 bei der Copolymerisation von Caprolacton und THF in Gegenwart von Wasser mit H₃PW₁₂O₄₀ als Katalysator anfallende Polymerphase eingesetzt, die die folgende Zusammensetzung aufwies: Caprolacton (54,3%), Polyetherester (41,2%), H₃PW₁₂O₄₀ (4,2%), H₂O (0,3%).

200 g dieser Mischung wurden bei 60 °C und 20 mbar von insgesamt 104 g Wasser, THF und Caprolacton befreit. Es verblieb ein homogener Rückstand mit klarer, viskoser Konsistenz. Hierzu wurden 100 g wasserfreies THF gegeben, wobei der größte Teil der gelösten H₃PW₁₂O₄₀ ausfiel. Nach Abtrennung von 6,8 g H₃PW₁₂O₄₀ durch Filtration wurde die Polymerphase mit 20 g Aktivkohle (Merck) versetzt und 4 Stunden bei Raumtemperatur geschüttelt. Im nach Abtrennung der Aktivkohle und Einengung im Vakuum erhaltenen Copolymer von THF und Caprolacton lag der H₃PW₁₂O₄₀-Gehalt unter 1 ppm.

## Patentansprüche

1. Verfahren zur Abtrennung von Heteropolyverbindungen aus Gemischen, die Polyether, Polyester und/oder Polyetherester, eine protische Komponente und Heteropolyverbindungen enthalten, dadurch gekennzeichnet, daß die protische Komponente mit Hilfe eines Membran-Trennverfahrens oder durch Destillation, gegebenenfalls azeotrop zusammen mit gegebenenfalls vorhandenem unumgesetztem Monomer und/oder Lösungsmittel vollständig abgetrennt werden und die Heteropolyverbindungen danach durch Zugabe des besagten Monomers in im wesentlichen reiner Form ausgefällt wird.

2. Verfahren nach Anspruch 1, dadurch gekennzeichnet, daß ein Polyoxytetramethylen-Gruppen enthaltender Polyether eingesetzt wird.

3. Verfahren nach Anspruch 1 oder 2, dadurch gekennzeichnet, daß als zugesetztes, im wesentlichen reines Tetrahydrofuran verwendet wird.

4. Verfahren nach einem der vorstehenden Ansprüche, dadurch gekennzeichnet, daß der Druck bei der Destillation so gewählt wird, daß sich eine Destillationstemperatur von 50 bis 70°C ergibt.

5. Verfahren nach einem der vorstehenden Ansprüche, dadurch gekennzeichnet, daß das Monomer-Polymer-Gewichtsverhältnis nach Abtrennung der protischen Komponente und gegebenenfalls Zugeben von besagtem im wesentlichen reinem Monomer 0,1:1 bis 10:1 beträgt.

6. Verfahren nach einem der vorstehenden Ansprüche, dadurch gekennzeichnet, daß die Polymerlösung nach Entfernung der protischen Komponente und Abtrennung der Heteropolyverbindung mit einem festen Adsorbens, das Heteropolyverbindungen adsorbieren kann, in Kontakt gebracht wird.

7. Verfahren nach einem der vorstehenden Ansprüche, dadurch gekennzeichnet, daß man als festes Adsorbens eines oder eine Mischung mehrerer aus der Gruppe Aktivkohlen, Aluminiumoxide, Erdalkalimetall- und Seltenerdmetalloxide, -hydroxide und -carbonate und basische Ionenaustauscher eingesetzt.

8. Verfahren nach einem der vorstehenden Ansprüche, dadurch gekennzeichnet, daß die abgetrennte protische Komponente zusammen mit ebenfalls abgetrenntem Monomer und/oder Lösungsmittel bei der Polymerisation wiederverwendet wird.

9. Verfahren nach einem der vorstehenden Ansprüche, dadurch gekennzeichnet, daß die nach Entfernung der protischen Komponente abgetrennte Heteropolyverbindungen als Polymerisationskatalysator wiederverwendet wird.

## Claims

1. A method of separating heteropoly compounds from mixtures comprising polyethers, polyesters and/or polyether esters, a protic component and heteropoly compounds, which method comprises completely separating the protic component with the aid of a membrane separation technique or by distillation, if desired azeotropically together with any solvent and/or unreacted monomer present and subsequently precipitating the heteropoly compounds by addition of said monomer in substantially pure form.

2. A method as claimed in claim 1, wherein a polyether is employed which includes polyoxytetramethylene groups.

3. A method as claimed in claim 1 or 2, wherein the added, substantially pure monomer used is tetrahydrofuran.

4. A method as claimed in any of the preceding claims, wherein the pressure during distillation is chosen so as to give a distillation temperature of from 50 to 70°C.

5. A method as claimed in any of the preceding claims, wherein the monomer/polymer weight ratio, after the protic component has been separated off and, if appropriate, said substantially pure monomer has been added, is from 0.1:1 to 10:1.

6. A method as claimed in any of the preceding claims, wherein after the protic component has been removed and the heteropoly compound has been separated off the polymer solution is brought into contact with a solid adsorbent capable of adsorbing heteropoly compounds.

7. A method as claimed in any of the preceding claims, wherein a solid adsorbent or a mixture of two or more solid adsorbents from the group consisting of activated carbons, aluminas, alkaline earth metal and rare earth metal oxides, hydroxides and carbonates, and basic ion exchangers is employed.

8. A method as claimed in any of the preceding claims, wherein the protic component separated off is reused in the polymerization together with solvent and/or monomer likewise separated off.

9. A method as claimed in any of the preceding claims, wherein the heteropoly compounds separated after removal of the protic component are reused as polymerization catalyst.

## Revendications

1. Procédé de séparation d'hétéropolycomposés à partir de mélanges contenant des polyéthers, des polyesters et/ou des polyétheresters, un constituant protique et des hétéropolycomposés, caractérisé en ce que le constituant protique est entièrement séparé à l'aide d'un procédé de séparation par membrane ou par distillation, éventuellement de façon azéotrope avec du monomère et/ou du solvant éventuellement présents, les hétéropolycomposés étant ensuite précipités par addition du monomère susmentionné sous forme quasiment pure.

2. Procédé selon la revendication 1, caractérisé en ce que l'on ajoute un polyéther contenant des groupements polyoxytétraméthylène.

3. Procédé selon la revendication 1 ou 2, caractérisé en ce que l'on utilise du tétrahydrofurane quasiment pur en tant qu'ajout.

4. Procédé selon l'une quelconque des revendications 1 à 3, caractérisé en ce que l'on choisit la pression lors de la distillation de sorte que la température de distillation s'élève à 50-70°C.

5. Procédé selon l'une quelconque des revendications 1 à 4, caractérisé en ce que le rapport en poids monomère/polymère après séparation du constituant protique et après l'éventuel ajout du monomère quasiment pur s'élève de 0,1 : 1 à 10 : 1.

6. Procédé selon l'une quelconque des revendications 1 à 5, caractérisé en ce que la solution de polymère est mise en contact avec un adsorbant solide pouvant adsorber des hétéropolycomposés, après élimination du constituant protique et séparation des hétéropolycomposés.

7. Procédé selon l'une quelconque des revendications 1 à 6, caractérisé en ce que l'on utilise un élément ou un mélange de plusieurs éléments choisis dans le groupe constitué par le charbon actif, les oxydes d'aluminium, les oxydes, hydroxydes et carbonates de métaux alcalino-terreux et de terres rares et les échangeurs d'ions basiques, en tant qu'adsorbant solide.

8. Procédé selon l'une quelconque des revendications 1 à 7, caractérisé en ce que l'on réutilise le constituant protique séparé avec le monomère et/ou le solvant éventuellement séparé, lors de la polymérisation.

9. Procédé selon l'une quelconque des revendications 1 à 8, caractérisé en ce que les hétéropolycomposés obtenus après élimination du constituant protique sont réutilisés en tant que catalyseurs de polymérisation.
